# EUROPEAN PATENT SPECIFICATION

(11) **EP 2 174 679 B1**
(45) Date of publication and mention of the grant of the patent: **27.03.2013**
(21) Application number: 09172513.5
(22) Date of filing: 08.10.2009
(51) Int. Cl.: A61M 5/145

(54) **Infusion device for drugs**
Infusionsgerät für Medikamente
Appareil de perfusion pour médicaments

(30) Priority: 09.10.2008 IT TO20080742
(43) Date of publication of application: 14.04.2010
(73) Proprietor: Cane' S.p.A., 10098 Rivoli (TO) (IT)
(72) Inventor: Cané, Mario, 10095, Collegno (TO) (IT)
(74) Representative: Robba, Pierpaolo

(56) References cited:
- EP-A1- 0 567 186
- EP-A1- 1 078 643
- DE-A1-102004 052 628
- US-A- 4 417 889

## Description

### Technical field

The present invention relates to an infusion device for drugs.

More precisely, the invention concerns a compact electromechanical device for infusion of drugs, contained in a syringe, a vial or a similar container, into the body of a patient into which a needle connected to the syringe through a cannula is introduced.

### Prior art

In the medical field, drug infusion devices, more commonly referred to as "infusion pumps", are often used for administering liquid drugs through a needle introduced into the patient's body, typically into a vein or under the skin.

As therapies requiring prolonged drug infusion are becoming increasingly diffused, even for patients who are not confined to bed, the need has arisen to provide compact, battery-powered infusion devices, which can be easily worn by the patient.

The prior art devices of the above kind generally comprise a drive unit, e.g. an electric motor, capable of imparting a rotary motion to a shaft, and a mechanical assembly converting the rotary motion imparted by the motor into a forward push linear motion.

Said forward push linear motion is advantageously exploited to cause the sliding of the piston of a syringe filled with drug and stably but removably associated with the device, so as to achieve drug infusion through a cannula connected to the needle introduced into the patient's body.

Utility model IT-U-193915 and European Patent EP 1078643, which are both in the name of the Applicant, disclose examples of drug infusion devices of the above kind.

However, most of the prior art infusion devices are not capable of causing suction of a drug into the syringe associated with the device.

Therefore, in such devices, the syringe has to be filled with the drug prior to being associated with the device. In some cases, the syringe is already filled in advance by the pharmaceutical company producing the drug, and in such cases a "pre-filled vial" is referred to, or the syringe is filled by the patient or a carer, who manually aspirates the drug into the syringe by exploiting a rod which is associated with the syringe piston and is subsequently removed in order to be replaced by the pusher of the infusion device.

The above discussion makes it apparent that, by the above devices, it is for instance impossible to obtain multiple consecutive infusions without removing the syringe from the device. Such consecutive infusions become for instance necessary when, at the end of a first drug infusion automatically performed with the device, the syringe is to be washed to remove possible drug residuals, which however are to be infused into the patient for reasons of cost or therapy completeness.

Moreover, it is impossible to obtain the automated syringe filling by using the same device as used for the infusion, and this, in case of patients exhibiting difficulties in handling objects, requires the presence of a carer who provides for the manual filling of the syringe.

DE 10-2004-052628 on which the preamble of claim 1 is based discloses an example of conventional infusion device capable of aspirating the drug into the syringe by making the motor move in opposite direction to the infusion direction.

As known, drug infusion devices must meet very severe reliability and safety standards, since operation errors could have even very serious consequences on the health of the patient undergoing the therapy, and thus the mere reversal of the motion of the motor does not ensure the possibility of using the device also for aspirating the drug.

Thus, it is a first object of the present invention to provide a drug infusion device that allows aspirating the drug into the syringe, is reliable and robust and is capable to keep the operation specifications unchanged over at last a few years.

It is a further object of the invention to provide a device which is cheap and easy to be constructed and therefore can be industrially manufactured at advantageous costs.

The above and other objects of the invention are achieved by means of a drug infusion device as claimed in the appended claims.

### Description of the invention

Advantageously, in accordance with the invention, the drug infusion device is programmed to impart also a backward push linear motion to the pusher, and therefore it includes a sliding element reducing the friction to the sliding of the contacting surfaces when a resistance contrasting such a backward push linear motion is present.

Thanks to the above features, the device according to the invention is capable of causing the suction of a drug into a syringe when the latter is already stably associated with the device itself.

Advantageously, in this manner, it is possible to effect multiple consecutive drug infusions, without removing the syringe from the device.

Moreover, advantageously, still thanks to the arrangement according to the invention, the syringe filling by patients exhibiting difficulties in handling objects becomes possible even in the absence of a carer.

Advantageously, still in accordance with the invention, said sliding element includes a ball or roller thrust bearing that is reliable and robust and is capable of meeting the safety criteria demanded for the construction of drug infusion devices.

### Brief Description of the Figures

A description of some preferred embodiments of the invention, given only by way of non-limiting examples, will be provided hereinbelow, with reference to the accompanying drawings in which:
- Fig. 1 is a side elevation of the drug infusion device according to a preferred embodiment of the invention;
- Fig. 2 is a longitudinal sectional view of the device shown in Fig. 1;
- Fig. 3 is an enlarged view of a portion of Fig. 2;
- Fig. 4 is a longitudinal sectional view of the device shown in Fig. 1, according to a variant embodiment;
- Fig. 5 is a longitudinal sectional view of a prior art device.

### Description of a Preferred Embodiment

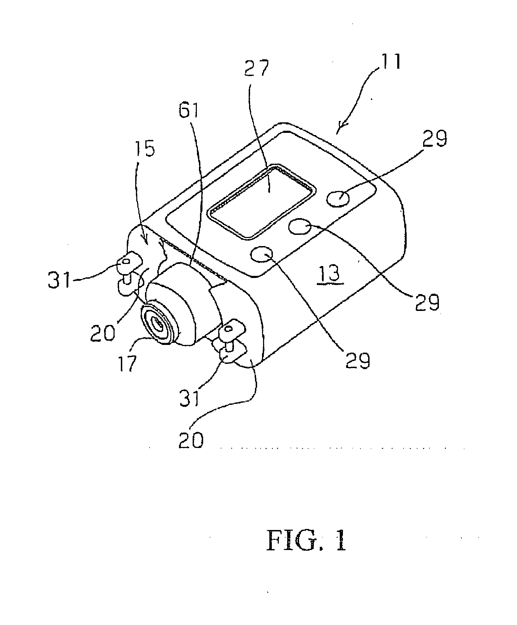

Referring to Figs. 1 to 3, there is shown a drug infusion device according to a preferred embodiment of the invention.

Said device, generally denoted in the Figures by reference numeral 11, includes a casing 13 with an elongated shape and a compact size, comparable to the size of a cigarette box, a ferrule 15, closing one of the bases of the casing, and a pusher 17, which is axially movable since it is associated with a sliding rod 19. The latter is partially received within casing 13 and projects therefrom through an opening 21 provided in ferrule 15.

Two radial seats 20, diametrically opposite relative to opening 21, are formed on ferrule 15 and are intended to receive radial tabs 23 of syringe 25, which is provided with a sliding piston 63 that is acted upon by pusher 17 when said syringe is to be stably fitted onto the device.

Said casing 13 further includes a display 27, some keys 29 for controlling the different functions for which the device is programmed, and a pair of eyelets 31 for insertion of a strap (not shown) facilitating the transportation of the device during use, for instance hung on the patient's neck.

The driving members causing the axial sliding of sliding rod 19 forth and back, in the directions shown by arrows Fw and Bw, respectively, are housed within casing 13.

Said driving members include an electric motor 35, equipped with a pinion 37 mounted on motor shaft 33 and engaging the teeth of a toothed crown gear 39. The crown gear is stably associated with a nut 41, which has an axial internal thread and through which a sliding rod 19 passes, which is externally threaded at least in correspondence of the portion engaging the thread of nut 41.

Motor 35 is stably associated with a supporting ferrule 43 substantially parallel to closing ferrule 15 and its operation is electronically controlled by a programmable electronic controller (not shown).

Toothed crown gear 39 has a plurality of axial holes 45 and defines, together with an optical detector 47 and a light emitter 49 both operating for instance in the infrared range, a corresponding position encoder thanks to which the signal representative of the angular position of toothed crown gear 39 and, correspondingly, of nut 41 rigidly connected therewith, is transmitted to the programmable controller controlling electric motor 35.

A radial peg 51 is further provided at the end of sliding rod 19 located inside casing 13 and said peg is slidable inside an axial groove 53 provided in a cylindrical guide 55 in order to prevent the rotation of sliding rod 19 while allowing its axial sliding.

The rotary motion of motor 35, driven by the electronic controller, is imparted to nut 41 through the coupling of pinion 37 with toothed crown gear 39.

Nut 41, by rotating about its axis, causes the axial sliding of sliding rod 19 thanks to the cooperation between the threads of the nut and the rod, rotation of the latter being prevented by peg 51 slidable in axial groove 53 of cylindrical guide 55.

Depending on the rotation direction imparted to motor 35 by the controller, for instance by inverting the power supply polarity, the forward or backward axial sliding of sliding rod 19, and consequently of pusher 17 associated therewith, in the direction of arrows Fw and Bw, respectively, will be obtained.

In the described arrangement, during the operation of the device for drug infusion, the resistance contrasting the forward motion of pusher 17 and caused for instance by the resistance encountered by the liquid drug to come out from cone 57 of syringe 25, is transmitted to sliding rod 19, whose forward axial sliding is therefore slowed down and which causes, as a reaction, the axial backward motion of nut 41 against supporting ferrule 43 in the direction shown by arrow Bw.

Such a phenomenon gives rise to an undesired friction increase in the mechanical components causing the forward motion of pusher 17, and a greater effort by the motor, which at worst can even become blocked. Blocking of the motor is signalled by encoder 47, 49, which stops the device by cutting off the power supply to the motor and signals, generally by means of an acoustic alarm signal, the occurrence of a probable occlusion along the drug flow line.

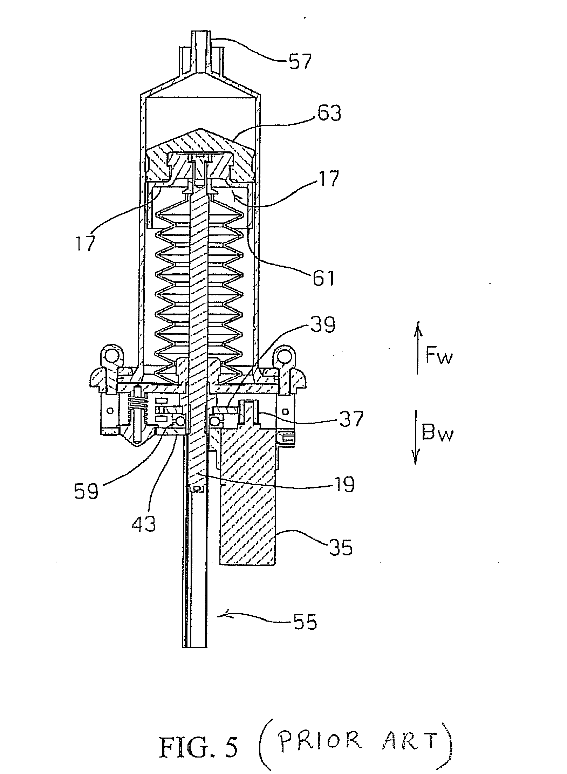

Referring to Fig. 5, which shows a device made in accordance with the prior art, in order to obviate this drawback a sliding element 59, consisting preferably of a ball thrust bearing or the like and aiming at reducing the friction between the contacting surfaces, is provided between supporting ferrule 43 and toothed crown gear 39.

Thanks to such an expedient, the resistance encountered during infusion is discharged onto thrust bearing 59 and substantially only an actual occlusion along the drug flow line can make the motor stop.

Once the syringe is removed from the device at the end of the infusion, in the prior art devices sliding rod 19 is brought back to the initial "zero" position, i.e. the position corresponding to the configuration shown in Fig. 1, where pusher 17 abuts with its peripheral edge 61 against ferrule 15 fastening the syringe, and the motor is made to rotate in opposite direction to the drug infusion direction. In this configuration, the device is again ready to receive a new syringe filled with the drug to be infused.

The initial "zero" position is determined when encoder 47, 49 signals the stop of toothed crown gear 39 for a predetermined time interval, the stop being determined by pusher 17 arriving into abutment against ferrule 15 with its peripheral edge 61.

During such a time interval, however short it is, motor 35 continues applying its rotation torque onto pinion 37 and can consequently cause pusher 17 to become blocked against ferrule 15: due to the elastic deformation caused on the contacting parts, it could become impossible to make the pusher further move forward in the forward direction for drug infusion.

In order to obviate this drawback, according to the prior art, electric motor 35 is supplied with two different voltages depending on whether it is to move pusher 17 forward or backward.

More particularly, motor 35 is supplied at the nominal voltage, ensuring the maximum torque, when the pusher 17 is to be moved forward in the direction of arrow Fw to effect drug infusion, whereas it is fed with a lower voltage, for instance half the nominal voltage (i.e. it is undersupplied) when it is merely to bring pusher 17 back to the initial zero position.

Thanks to such an expedient, the motor is no longer capable of causing pusher 17 to become blocked against ferrule 15, since the maximum torque that can be applied by motor 35 is reduced.

Yet, it is apparent that, due to the described arrangement, the prior art infusion devices are not capable of causing suction of a drug into the syringe, should the latter be associated with the device while the pusher 17 is moving backwards.

Actually, the torque available at the motor pinion would not be sufficient to allow overcoming the resistance put up, for instance by the conical nozzle, to the entrance of the drug into the syringe and to the sliding of syringe piston 63 in which pusher 17 is firmly engaged.

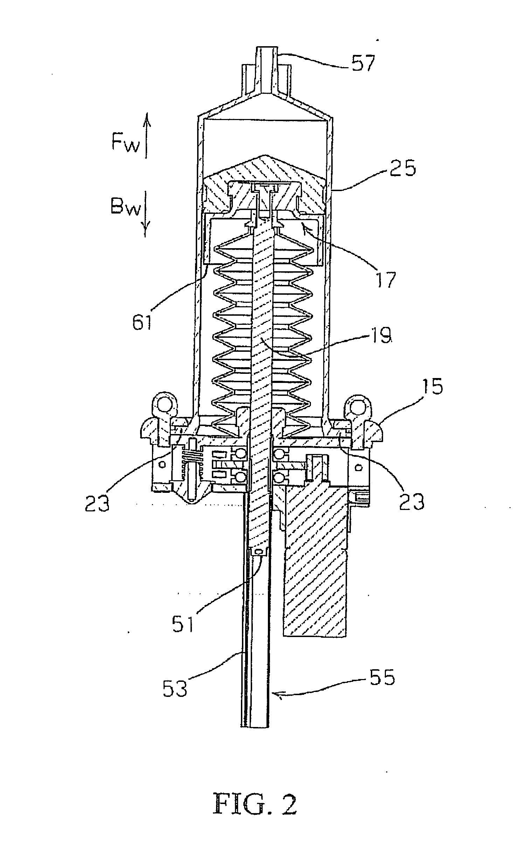

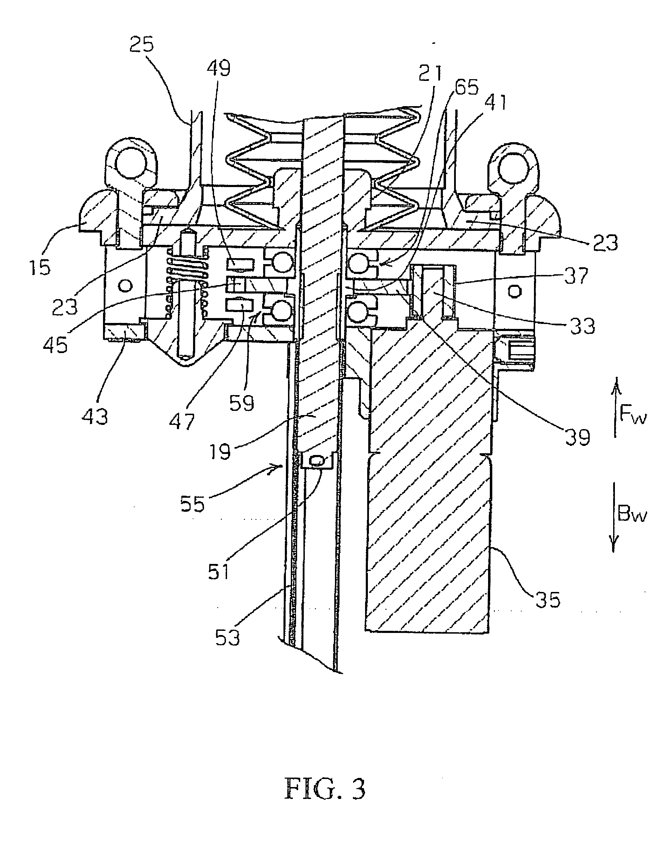

Turning back to Figs. 2 and 3, in accordance with the invention, drug infusion device 11 is therefore equipped with a sliding element 65, preferably consisting of a ball thrust bearing or the like, onto which the axial forces due to the resistance encountered in the drug suction phase are discharged. Said member 65 is preferably arranged between ferrule 15 fastening the syringe and toothed crown gear 39 imparting the axial motion to sliding rod 19.

Thanks to such an expedient, the resistance, if any, put up by the fluid entering a syringe associated with the device while the pusher is moving backwards, is axially discharged onto sliding element 65 without the occurrence of a substantial increase in the friction between members moving relative to each other. Thus, motor 35 can advantageously be supplied so as to provide the maximum available torque, or anyway a torque close to such a maximum torque, also while rod 19 is moving backwards, so that use of the device for suction of a drug into the syringe is made possible.

To this aim, in accordance with the invention, the programmable electronic controller controlling the operation of electric motor 35 can be advantageously programmed to cause also the drug suction into the syringe fit onto the device, besides the drug infusion from the syringe fit onto the device.

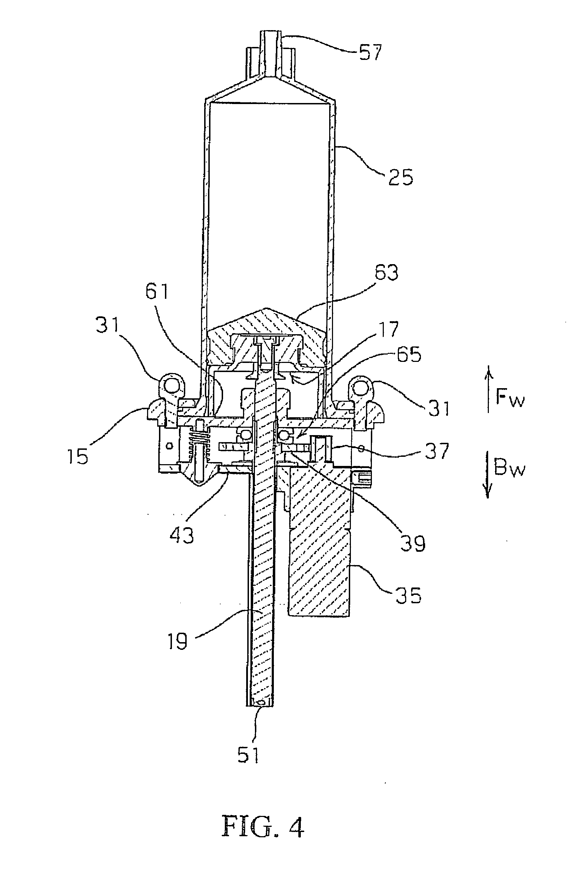

Turning now to Fig. 4, there is shown a variant embodiment of the drug infusion device according to the invention, when such a device is in the zero position at the end of the drug suction into syringe 25 fit thereon.

In such a variant embodiment, a single sliding element 65 is provided, which preferably consists of a corresponding thrust bearing and intervenes during the phase of suction of the liquid into the syringe fit on the device.

Indeed, it is known that in some applications the resistance encountered by motor 35 during infusion is lower than that encountered during suction.

It is therefore clear that, under such circumstances, providing the device with a sliding element operating during infusion can be superfluous, whereas the provision of a sliding element aimed at eliminating the friction originating during aspiration will be still necessary.

The invention can undergo several changes and modifications, all lying within the same inventive principle, and its scope is only defined by the claims.

## Claims

1. A drug infusion device (11), including a casing (13), a ferrule (15) having coupling means (20) for receiving a syringe (25) equipped with a sliding piston (63), and a pusher (17) that is axially movable to make said piston (63) slide in the syringe, said pusher being associated with a sliding rod (19) that is partially received within the casing (13) and projects therefrom through an opening (21) provided in the ferrule (15), said casing (13) housing driving members causing the axial sliding of the sliding rod (19), said driving members including an electric motor (35) electronically controlled by a programmable electronic controller and programmed to cause the infusion of a drug from the syringe fit onto the device and/or the suction of a drug into the syringe fit onto the device, said driving members further including a toothed crown gear (39) stably associated with a not (41), which has an axial internal thread and through which said sliding rod (19) passes, said sliding rod (19) being externally threaded at least in correspondence of the portion engaging the axial internal thread of the nut (41), the drug infusion device (11) being **characterised in that** it includes a sliding element (65) comprising a ball or roller thrust bearing arranged between the ferrule (15) and the toothed crown gear (39) imparting the axial motion to sliding rod (19), onto which the axial resistance, if any, put up by the fluid entering a syringe associated with the device is discharged while the pusher (17) is moving backwards during drug suction, thereby preventing the occurrence of a substantial increase in the friction between members moving relative to each other.

2. The device as claimed in claim 1, wherein said driving members further include a pinion (37) associated with the motor shaft (33) and engaging the teeth of the toothed crown gear (39).

3. The device as claimed in claim 1 or 2, wherein the motor (35) can be supplied so as to provide the maximum available torque while the rod (19) is moving backwards, whereby use of the device for drug aspiration into the syringe is made possible.

4. The device as claimed in any preceding claim, wherein a second sliding element (59) is provided, onto which there is discharged the axial resistance, if any, put up by the fluid leaving a syringe associated with the device while the pusher (17) is moving forward during the drug infusion phase, without the occurrence of a substantial increase in the friction between members moving relative to each other.

5. The device as claimed in claim 4, wherein said second sliding element (59) is a ball thrust bearing or the like.

6. The device as claimed in claim 5, wherein the motor (35) is stably associated with a supporting ferrule (43) substantially parallel to the ferrule (15) onto which the syringe is fit, and wherein said second thrust bearing (59) is arranged between the supporting ferrule (43) and the toothed crown gear (39),

7. The device as claimed in claim 1, wherein the toothed crown gear (39) has a plurality of axial holes (45) and defines, together with an optical detector (47) and a light emitter (49), a corresponding position encoder thanks to which the signal representative of the angular position of the toothed crown gear (39) and, correspondingly, of the nut (41) firmly connected therewith, is transmitted to the programmable controller controlling the electric motor (35).

8. The device as claimed in any preceding claim, wherein the pusher (17) is firmly engaged in the syringe piston (63).

9. The device as claimed in any preceding claim, wherein said casing (13) has an elongated shape and a compact size, comparable to the size of a cigarette box.

10. The device as claimed in any preceding claim, wherein two radial seats (20), diametrically opposite relative to opening (21), are formed on ferrule (15) and are intended to receive radial tabs (23) of a syringe (25).

11. The device as claimed in any preceding claim, wherein said casing (13) comprises a display (27) and some keys (29) for controlling the different functions for which the device is programmed.

12. The device as claimed in any preceding claim, wherein said casing (13) comprises a pair of eyelets (31) for insertion of a strap facilitating the transportation of the device during use.

13. The device as claimed in any preceding claim, wherein a radial peg (51) is Further provided at the end of sliding rod (19) located inside casing (13) and said peg is slidable inside an axial groove (53) provided in a cylindrical guide (55) in order to prevent the rotation of sliding rod (19) while allowing its axial sliding.

## Patentansprüche

1. Medikamenteninfusionsgerät (11) umfassend ein Gehäuse (13), eine Hülse (15) mit Kupplungsmitteln (20) zur Aufnahme einer mit einem verschiebbaren Kolben (63) versehenen Spritze (25), und einen Schieber (17), der zum Verschieben des verschiebbaren Kolbens (63) in der Spritze axial beweglich ist, wobei der Schieber mit einer Schiebestange (19) verbunden ist, die in dem Gehäuse (13) teilweise aufgenommen ist und durch eine an der Hülse (15) angeordnete Öffnung (21) aus diesem herausragt, wobei das Gehäuse (13) Antriebsorgane zum Betätigen der Axialverschiebung des verschiebbaren Kolbens (19) aufnimmt, wobei die Antriebsorgane einen Elektromotor (35) umfassen, der durch eine programmierbare, elektronische Steuerung elektronisch gesteuert ist und zum Betätigen der Infusion eines Medikaments aus der an dem Gerät angebrachten Spritze und/oder zum Betätigen des Ansaugens eines Medikaments in die an dem Gerät angebrachte Spritze programmiert wird, wobei die Antriebsorgane weiter einen Zahnkranz (39) umfassen, der mit einer ein Axialinnengewinde aufweisenden Mutter (41) fest verbunden ist, durch die die Schiebestange verläuft, wobei die Schiebestange (19) mit einem Außengewinde zumindest im Bereich des in das Axialinnengewinde der Mutter (41) eingreifenden Abschnitts ausgestattet ist, wobei das Medikamenteninfusionsgerät (11) **dadurch gekennzeichnet ist, dass** es ein Schiebeelement (65) umfasst, das einen zwischen der Hülse (15) und dem der Schiebestange (19) die Axialbewegung erteilenden Zahnkranz (39) angeordneten Kugeldrucklager oder Rollendrucklager aufweist und auf das der eventuelle axiale Widerstand abgeführt wird, der durch das in eine dem Gerät zugeordnete Spritze eintretende Medium geleistet wird, während sich der Schieber (17) beim Ansaugen des Medikaments zurückbewegt, wodurch das Auftreten einer erheblichen Erhöhung der Reibung zwischen sich relativ zueinander bewegenden Teilen vermieden wird.

2. Gerät nach Anspruch 1, wobei die Antriebsorgane weiter ein Ritzel (37) umfassen, das der Motorwelle (33) zugeordnet ist und in das Zähne des Zahnkranzes (39) eingreifen.

3. Gerät nach Anspruch 1 oder 2, wobei der Motor (35) so versorgt werden kann, dass er das maximal erreichbare Drehmoment erzeugt, wenn sich die Schiebestange (19) zurückbewegt, wodurch die Benutzung des Geräts zum Ansaugen des Medikaments in die Spritze ermöglicht wird.

4. Gerät nach einem der vorhergehenden Ansprüche, wobei ein zweites Schiebelement (59) vorgesehen ist, auf das der eventuelle axiale Widerstand abgeführt wird, der durch das aus einer dem Gerät zugeordneten Spritze austretende Medium geleistet wird, wenn sich der Schieber (17) bei der Medikamenteninfusionsschritt zurückbewegt, ohne dass eine erheblichen Erhöhung der Reibung zwischen sich relativ zueinander bewegenden Teilen stattfindet.

5. Gerät nach Anspruch 4, wobei das zweite Schiebeelement (59) ein Kugeldrucklager oder dergleichen ist.

6. Gerät nach Anspruch 5, wobei der Motor (35) mit einer Traghülse (43) fest verbunden ist, die im Wesentlichen parallel zu der Hülse (15) ist, an der die Spritze angebracht ist, und wobei das zweite Drucklager (59) zwischen der Traghülse (43) und dem Zahnkranz (39) angeordnet ist.

7. Gerät nach Anspruch 1, wobei der Zahnkranz (39) mehrere Axialbohrungen (45) aufweist und, zusammen mit einem optischen Detektor (47) und einem Lichtsender (49), einen entsprechenden Positionsgeber bildet, mit dessen Hilfe das Signal, das die Winkelposition des Zahnkranzes (39) und dementsprechend der mit ihm fest verbundenen Mutter (41) darstellt, an die den Elektromotor steuernde, programmierbare Steuerung versendet wird.

8. Gerät nach einem der vorhergehenden Ansprüche, wobei der Schieber (17) fest im Eingriff in dem Spritzenkolben (63) steht.

9. Gerät nach einem der vorhergehenden Ansprüche, wobei das Gehäuse (13) eine längliche Form sowie kompakte Maße hat, die mit jenen einer Zigarettenschachtel vergleichbar sind.

10. Gerät nach einem der vorhergehenden Ansprüche, wobei zwei der Öffnung (21) diametral gegenüberliegende Radialaufnahmen (20) an der Hülse (15) angeformt sind und dazu bestimmt sind, radiale Flügel (23) einer Spritze (25) aufzunehmen.

11. Gerät nach einem der vorhergehenden Ansprüche, wobei das Gehäuse (13) einen Bildschirm (27) sowie einige Tasten (29) zum Steuern der verschiedenen Funktionen umfasst, für die das Gerät programmiert ist.

12. Gerät nach einem der vorhergehenden Ansprüche, wobei das Gehäuse (13) ein Paar von Ösen (31) zum Hindurchführen eines den Transport des Geräts bei Benutzung erleichternden Gurtes umfasst.

13. Gerät nach einem der vorhergehenden Ansprüche, wobei weiter ein radialer Stift (51) an dem in dem Gehäuse (13) befindlichen Ende der Schiebestange (19) angeordnet ist und dieser Stift in einer an einer zylindrischen Führung (55) vorgesehenen Nut (53) verschiebbar ist, um eine Verdrehung der Schiebestange (19) zu verhindern und die Axialverschiebung derselben zu erlauben.

## Revendications

1. Appareil de perfusion pour médicaments (11), comprenant un boîtier (13), une bague (15) pourvue de moyens de couplage (20) destinés à recevoir une seringue (25) équipée d'un piston coulissant (63), et un pousseur (17) qui est mobile axialement pour faire coulisser ledit piston (63) dans la seringue, ledit pousseur étant associé à une tige coulissante (19) qui est partiellement reçue dans le boîtier (13) et qui sort de celui-ci à travers une ouverture (21) prévue dans la bague (15), ledit boîtier (13) logeant des éléments d'entraînement qui provoquent le coulissement axial de la tige coulissante (19), lesdits éléments d'entraînement comprenant un moteur électrique (35) contrôlé électroniquement par une unité de contrôle électronique programmable et programmé pour provoquer l'infusion d'un médicament à partir de la seringue montée sur l'appareil et/ou l'aspiration d'un médicament dans la seringue montée sur l'appareil, lesdits éléments d'entraînement comprenant en outre une couronne dentée (39) associée de manière stable avec un écrou (41), qui est pourvu d'un filetage interne axial et à travers lequel ladite tige coulissante (19) passes, ladite tige coulissante (19) étant extérieurement filetée au moins en correspondance de la partie engageant le filetage interne axial de l'écrou (41), ledit appareil de perfusion pour médicaments (11) étant **caractérisé en ce qu'**il comprend un élément coulissant (65) comportant un palier de butée à billes ou à rouleaux agencé entre la bague (15) et la couronne dentée (39) qui confère le mouvement axial à la tige coulissante (19), sur lequel l'éventuelle résistance axiale mise en place par le fluide qui entre dans une seringue associée à l'appareil est déchargée lorsque le pousseur (17) se déplace vers l'arrière lors de l'aspiration d'un médicament, empêchant ainsi l'apparition d'une augmentation importante du frottement entre éléments se déplaçant l'un par rapport à l'autre.

2. Appareil selon la revendication 1, dans lequel lesdits éléments d'entraînement comprennent en outre un pignon (37) associé à l'arbre moteur (33) et en prise avec les dents de la couronne dentée (39).

3. Appareil selon la revendication 1 ou 2, dans lequel le moteur (35) peut être alimenté de manière à fournir le couple maximum disponible quand la tige (19) se déplace vers l'arrière, de telle sorte que l'utilisation du dispositif pour l'aspiration de médicaments dans la seringue est rendue possible.

4. Appareil selon l'une quelconque des revendications précédentes, dans lequel un deuxième élément coulissant (59) est prévu, sur lequel l'éventuelle résistance axiale mise en place par le fluide qui sort d'une seringue associée à l'appareil est déchargée lorsque le pousseur (17) se déplace vers l'avant lors de la phase de perfusion d'un médicament, sans l'apparition d'une augmentation importante du frottement entre éléments se déplaçant l'un par rapport à l'autre.

5. Appareil selon la revendication 4, dans lequel ledit deuxième élément coulissant (59) est un palier de butée à billes ou similaire.

6. Appareil selon la revendication 5, dans lequel le moteur (35) est associé de manière stable à une bague de support (43) sensiblement parallèle à la bague (15) sur laquelle la seringue est montée, et dans lequel ledit deuxième palier de butée (59) est agencé entre la bague de support (43) et la couronne dentée (39).

7. Appareil selon la revendication 1, dans lequel la couronne dentée (39) comporte une pluralité de trous axiaux (45) et définit, conjointement avec un détecteur optique (47) et un émetteur de lumière (49), un correspondant codeur de position grâce auquel le signal représentatif de la position angulaire de la couronne dentée (39) et, de manière correspondante, de l'écrou (41) solidement connecté à celle-ci, est transmis à l'unité de contrôle programmable qui contrôle le moteur électrique (35).

8. Appareil selon l'une quelconque des revendications précédentes, dans lequel le pousseur (17) est solidement engagé dans le piston (63) de la seringue.

9. Appareil selon l'une quelconque des revendications précédentes, dans lequel ledit boîtier a une forme allongée et une taille compacte, comparable à celle d'un paquet de cigarettes.

10. Appareil selon l'une quelconque des revendications précédentes, dans lequel deux logements radiaux (20), diamétralement opposés par rapport à l'ouverture (21), sont formés sur la bague (15) et sont destinés à recevoir des ailettes radiales (23) d'une seringue (25).

11. Appareil selon l'une quelconque des revendications précédentes, dans lequel ledit boîtier (13) comprend un écran (27) et quelques boutons (29) pour contrôler les différent fonctions pour lesquelles l'appareil est programmé.

12. Appareil selon l'une quelconque des revendications précédentes, dans lequel ledit boîtier (13) comprend une paire de fentes (31) pour l'insertion d'une sangle qui facilite le transport de l'appareil pendant son utilisation.

13. Appareil selon l'une quelconque des revendications précédentes, dans lequel une goupille radiale (51) est en outre prévue à l'extrémité située à l'intérieur du boîtier de la tige coulissante (19) et ladite goupille peut coulisser à l'intérieur d'une rainure axiale (53) agencée dans une guide cylindrique (55) pour empêcher la rotation de la tige coulissant (19) tout en permettant son glissement axial.
